# EUROPEAN PATENT APPLICATION

(11) **EP 2 339 026 A1**
(43) Date of publication of application: **29.06.2011**
(21) Application number: 09814132.8
(22) Date of filing: 13.08.2009
(51) Int. Cl.: C12Q 1/68

(54) **METHOD FOR SIMULTANEOUSLY DETECTING HISTOPLASMA CAPSULATUM AND PARACOCCIDIOIDES BRASILIENSIS**

(30) Priority: 19.09.2008 ES 200802665
(71) Applicant: Instituto de Salud Carlos III, 28029 Madrid (ES)
(72) Inventor: BUITRAGO SERNA, Mª José, E-28029 Madrid (ES); CUENCA-ESTRELLA SINDE, Manuel, E-28029 Madrid (ES); RODRIGUEZ-TUDELA, Juan Luis, E-28029 Madrid (ES); GOMEZ LOPEZ, Alicia, E-28029 Madrid (ES)
(74) Representative: Pons Ariño, Angel
(86) International application number: PCT/ES2009/070340
(87) International publication number: WO 2010/031888

(57) **Abstract**

The present invention relates to a method for simultaneously detecting DNA of *Histoplasma capsulatum* and *Paracoccidioides brasiliensis* by means of the real-time quantitative multiplex PCR technique, based on specific probes marked with different fluorophores. This technique is applicable both in biological samples and microbiological cultures and environmental samples. Furthermore, another aspect of the invention is that a kit has been developed enabling the two micro-organisms to be detected simultaneously.

## Description

The present invention relates to a method for simultaneously detecting DNA of *Histoplasma capsulatum* and *Paracoccidioides brasiliensis* by means of the real-time quantitative multiplex PCR technique, based on specific probes marked with different fluorophores. This technique is applicable both in biological samples and microbiological cultures and environmental samples. Furthermore, another aspect of the invention is that a kit has been developed enabling the two micro-organisms to be detected simultaneously.

### PRIOR ART

The microorganisms *Histoplasma capsulatum* and *Paracoccidioides brasiliensis* are the causal factors of the diseases known as histoplasmosis and paracoccidioidomycosis. These diseases are primary mycoses of frequent appearance in certain geographic areas, particularly in tropical and subtropical areas with temperate and humid climate. In these endemic areas, the appearance of AIDS has worsened the problem of primary mycoses until making it one of the most frequent pathologies. The prevalence of these diseases is increasing in Spain due to infections imported in travellers or emigrants from endemic areas, especially from the population from Latin America. According to the National Institute of Statistics (www.ine.es), 35% of the emigrant population residing in Spain comes from endemic areas and, furthermore, one million Spaniards travel each year to these countries.

In endemic areas, histoplasmosis is one of the most frequent opportunistic infections in patients with AIDS. Therefore, histoplasmosis must be included in the differential diagnosis of South American patients with AIDS, with signs of systemic infection and affection of the mononuclear-phagocytocic system. In the case of histoplasmosis in travellers or tourists, the disease manifests itself as a respiratory infection that may vary in terms of symptoms, from catarrhal symptoms to a serious infection that places the life of the patient at risk.

With respect to paracoccidioidomycosis, it is the most frequent systemic mycosis of Tropical America and, although infrequent in Europe, several cases have been described in recent years associated to people from endemic regions. Generally, these patients have a chronic form of the disease that may be unifocal affecting the lung or multifocal represented by a series of skin or mucosal lesions and affecting the lungs. It is also frequent that several years pass from acquiring the disease until developing the symptoms. Paracoccidioidomycosis must also be included in the differential diagnosis of patients from Latin America with a respiratory infection, whether skin lesions appear or not.

The diagnosis of both mycoses is complicated for which reason, to date, the certain diagnosis has been based on culture of the microorganism and later identification by means of microscopic techniques or exoantigens. Nevertheless, the culture may take 1 or 2 weeks to be positive and the identification may be complex in centres without experience. The viewing of fungal structures can help to start the treatment, but does not confirm the diagnosis. The serological tests may help the diagnosis, but they have a limited specificity in endemic areas and a low sensitivity in immunodepressed patients. On the other hand, in the case of histoplasmosis, there is a technique to detect the antigen in the urine, which is highly reliable, even in immunodepressed patients. But, to date, this technique has not been marketed and is only available in benchmark centres in the USA.

Faced with this situation, the Mycology Service of the National Microbiology Centre has developed the process described in this invention to simultaneously detect both fungal pathogens since the clinical analysis is similar in both and the population that may be affected by said pathogens is the same.

The method described in the present invention provides a tool that resolves some of the problems presented by other methods, since it significantly reduces the time used by conventional diagnosis methods (reduced from 1-2 weeks to 1-2 days) and serves to simultaneously detect both microorganisms *Histoplasma capsulatum* and *Paracoccidioides brasiliensis.* Likewise, the speed in the detection makes it possible to start treatment of histoplasmosis and/or of paracoccidioidomycosis earlier, improving the prognosis of patients and enabling monitoring response to the treatment.

### EXPLANATION OF THE INVENTION

The present invention discloses a method for simultaneously detecting DNA of *Histoplasma capsulatum* and *Paracoccidioides brasiliensis* by means of the real-time quantitative multiplex PCR technique, based on specific probes of molecular beacon *type* marked with two different fluorophores.

Molecular beacons are probes that have a fork or loop shape, incorporating an emitter fluorophore and a quencher, which inhibits the emission of fluorescence whilst the probe does not hybridize. When the probe binds to the DNA, it forms a rigid, double-strand helix, whereby the probe loses its fork shape, separating the emitter from the quencher, which causes the detectable fluorescence. Molecular beacons are formed by 10-30 base pairs, they allow the detection of changes of a single base and they are probes especially indicated for use in multiplex PCR assays since they can be marked with different fluorophores and they have different thermodynamic properties that favour high specificity.

The method described in the present invention has a series of advantages with respect to the conventional techniques:
1. Reliability and simplicity to identify *Histoplasma* and *Paracoccidioides* under culture.
2. In biological samples, the technique shows a sensitivity greater than that of the serological tests.
3. As regards samples related to the respiratory apparatus, it has a sensitivity comparable with that of identification by culture.
4. The PCR techniques have a specificity of 100%, since they do not amplify DNA from other fungal species.
5. Its use in biological samples makes it possible to reduce the diagnosis time from 10-14 days in identification by culture to 24-48 hours
6. The PCR technique applied to biological samples can be performed in installations with conventional biosecurity levels (level 2).

As regards innovative characteristics, the following can be highlighted:
1. The possibility of simultaneously detecting both pathogens.
2. Its high specificity, which avoids secondary steps such as the sequencing of the product or the display of marked DNA bands.
3. The significant reduction of the time used in detecting both pathogens.

In this sense, a first aspect of the present invention is a method for simultaneously detecting *Histoplasma capsulatum* and *Paracoccidioides brasiliensis* comprising:
a. obtaining a sample and isolating its DNA,
b. amplifying a fragment of the ITS region of the ribosomal DNA of *Histoplasma capsulatum* and/or *Paracoccidioides brasiliensis* contained in the DNA isolated in step (a),
c. determining the deviation of step (b) with respect to the controls. These controls may be positive controls and/or negative controls,
d. analysing the deviation of step (c) and attributing it to the presence of said microorganisms.

The term "simultaneous detection" is understood to be the capacity of this method to detect both the presence of *Histoplasma capsulatum* and *Paracoccidioides brasiliensis* in a sample and with a single real-time PCR reaction.

The isolation of the DNA sample is performed by means of known methods that make it possible to obtain the total DNA from the sample. In this way, if the sample contains *Histoplasma capsulatum* and/or *Paracoccidioides brasiliensis,* its DNA will be extracted and the fragments of interest of the ITS region will be amplified as a consequence of the method's high specificity.

The regions known as ITS (*Internal Transcribed Spacer*) are sequences of non-functional ribosomal DNA situated between sequences that code for subunits of ribosomal RNA 18S and 5.8S (ITS1) and between subunits 5.8S and 28S (ITS2). The ITS have a high degree of variation between phylogenetically close species, which makes it possible to use them specifically to determine the species as well as variations thereof.

The term "negative control" refers to the sample from people not infected with *Histoplasma capsulatum* and/or with *Paracoccidioides brasiliensis,* as well as the internal controls without DNA used in the PCR reactions to discard contaminations in the handling of samples. The term "positive control" refers to samples containing DNA from the microorganisms cited with total security.

A preferred embodiment of said method is that wherein, to carry out the amplification, the concentration of DNA is at least 10 fg/µl since this is an amount that represents the detection limit of the DNA fragments of *Histoplasma capsulatum* and *Paracoccidioides brasiliensis.*

In another preferred embodiment of the method, the sample may be chosen from a biological sample, a microbiological culture or an environmental sample.

The term "biological sample" refers to an isolated sample of materials of biological origin that may be of human, animal or plant origin. The main application of this method is aimed at samples of human origin but the method of the present invention may be applied to detect these microorganisms causing histoplasmosis and paracoccidioidomycosis in other living beings that may behave as transmission vehicles of said microorganisms. The biological sample may be selected from the list comprising respiratory sample, bone marrow aspirates, cerebrospinal fluid, tissue biopsy, urine or blood flow. Blood fluid is understood to be blood, serum or plasma.

The sample called "microbiological culture" relates to any type of culture of microorganisms performed in growth media, whether solid, liquid or those that allow the growth of the microorganisms.

The term "environmental sample" refers to any other type of sample from any medium that can contain *Histoplasma capsulatum* and *Paracoccidioides brasiliensis.* In other words, this last type of samples refers to samples that may come from soil, water, air or any other inorganic matter.

In another more preferred embodiment, the biological sample is related to the respiratory apparatus. A "sample related to the respiratory apparatus" is understood as a sample of biological origin of any tissue of the respiratory apparatus or fluid secreted in said tissues. Therefore, they are samples related to the respiratory apparatus, bronchial secretion, bronchoalveolar lavage, sputum or oropharyngeal exudate, without excluding any other type of sample included within the definition of this paragraph.

In another preferred embodiment, the amplification of the ITS2 region of the ribosomal DNA of *Histoplasma capsulatum* is performed by means of the primers SEQ ID NO: 1 and SEQ ID NO: 2 and the amplification of the ITS1 region of the ribosomal DNA of *Paracoccidioides brasiliensis* is performed by means of the primers SEQ ID NO: 4 and SEQ ID NO: 5.

In another preferred embodiment, the amplification is performed by means of real-time multiplex PCR characterized in that specific probes are used marked with two different fluorophores.

The specific probes consist of a sequence of linear and single-strand DNA, complementary, partially, of any part of the sequence of the amplified fragment corresponding to the ITS of *Histoplasma capsulatum* and *Paracoccidioides brasiliensis.*

The DNA amplification technique known as PCR (*Polymerase Chain Reaction*) is based on the repetition of a cycle formed by three stages:
Denaturing of the double-strand DNA achieved with high temperatures (94ºC in the majority of the cases, hybridization of zone 3' specific for each of the strands (the temperature is specific for each pair of primers) and extension of the primer due to action of the polymerase DNA (the time necessary for the extension depends on the fragment length).

The quantity of copies obtained after amplification of the fragments is proportional to the initial quantity thereof, i.e. to the quantity of micro-organisms present in the samples (more specifically, to the quantity of DNA extracted and the quality thereof).

PCR is called multiplex when it uses more than one pair of primers for simultaneously amplifying several DNA fragments in a single amplification reaction. To be able to perform these amplifications, both the design of the primers and the probes must be sufficiently specific to avoid cross-reactions that give rise to non-specific amplifications that may invalidate the method. This invention uses a combination of primers and probes that have shown themselves to be very effective in fragment amplification.

A primer is a short oligonucleotide that can be synthesized *in vitro* to be used in several molecular techniques. They are designed as complementary sequences of a region of target DNA that one wants to detect. To obtain a fragment, two primers are necessary, one of them will be linked to the mould strand (direct primer) and the other to the complementary strand (reverse primer), in a position that makes it possible to obtain, by means of successive amplifications with a heat-resistance polymerase DNA enzyme, a fragment that can be detected and/or quantified.

For primer design, it is necessary to make a series of predictions such as melting temperature (Tm) or the possibility of formation of forks that can reduce, by competition, the effectiveness of the hybridization with the sequence of target DNA. The variables to be borne in mind in primer design are fundamentally: Size of the oligonucleotide, melting temperature (Tm), specificity with the DNA of the ITS regions of the ribosomal DNAs of *Histoplasma capsulatum* and *Paracoccidioides brasiliensis* that are to be amplified, complementary sequences, contained in G/C and fragments of pyrimidines (T, C) or purines (A, G), 3' terminal sequence and 5' terminal sequence and central regions.

Furthermore, for the synthesis of DNA, the presence of triphosphate nucleotides dNTPs is necessary, i.e. dATP, dTTP, dCTP and dGTP, DNA heat-resistant polymerase, magnesium chloride in a determined concentration as well as buffers that maintain the suitable physicochemical characteristics for the functioning of all components and therewith the amplification is achieved.

The quantitative PCR technique or also known as real-time PCR makes it possible to quantify in real time the amplification of these fragments of interest. The method described in this invention uses two probes marked with a different fluorophore that is specifically bound to amplified DNA fragments, so that, each one of the probes hybridize with the corresponding fragment of DNA, present in the amplified sequences of the ITS. Each one of the probes is marked with a different fluorophore, in this way it simultaneously detects and quantifies, by fluorescence emission, the amplification of each one of the fragments. To detect the fluorescence from each one of the amplifications, all subjected to the same amplification conditions, the thermocycler has a detector coupled that measures the fluorescence in real time throughout the duration of the amplification cycles. To be able to know the concentration of DNA obtained as a product, it is necessary to use known concentration templates that make it possible to monitor the functioning of the technique.

Thus, a more preferred embodiment of the method is that wherein the probes used are:
a. SEQ ID NO: 3 (the first seven nucleotides are complementary to the seven last ones) for the amplified fragment of *Histoplasma capsulatum* and it is marked at the 5' end with a fluorophore and at the 3' end with a quencher, and
b. SEQ ID NO: 6 (the first six nucleotides are complementary to the six last ones) for the amplified fragment of *Paracoccidioides brasiliensis* and it is marked at the 5' end with a different fluorophore from that used in step (a) and at the 3' end with a quencher.

The probes used in this embodiment are of the molecular beacon type.

These probes form a hairpin due to the presence of 6-7 complementary nucleotides between one another at both ends of the sequence, whose secondary structure maintains the fluorophore and the quencher close, so that any photon emitted by the reporter is absorbed by the quencher. The rest of the sequence, formed by 19 nucleotides, situated between the complementary ends, is also complementary to a part of the sequence of the amplified fragments. When the probe hybridizes with the amplified DNA it opens and distances the fluorophore from the quencher, allowing fluorescence to be emitted that can be detected and quantified.

The term "quencher" refers to a deactivator or extinguisher of fluorescence, i.e. a molecule capable of absorbing photons emitted by the fluorophore and dissipating the energy in the form of heat, so that no emission of fluorescence occurs.

In an even more preferred embodiment, the fluorophore of step (a) is FAM (5 or 6-carboxyfluorescein), the fluorophore of step (b) is HEX (5'-hexachloro-fluorescein phosphoramidite) and the quencher is BHQ1.

Another aspect of the present invention is the method described in preceding paragraphs as a tool to monitor the response to a histoplasmosis and/or paracoccidioidomycosis treatment wherein serial samples are taken from patients who have received a treatment. The analysis of the rise or fall of the fungal DNA quantification makes it possible to assay if the response to the treatment is negative or positive, respectively.

The monitoring process comprises a series of steps that start by the taking of serial samples. Serial sampling is understood to be the extraction of any type of biological samples, including those mentioned in the invention. The sampling is performed at different times from when the treatment is administered, so that quantification of the amplification of the ITS fragments of the respective microorganisms in each one of the samples from the same patient will indicate the efficacy thereof. Thus, a decrease in the deviation of the amplification values respect to a control, the latter represented, for example, by amplification value in a same individual, prior to treatment, would mean that the treatment is having an effect on decreasing the level of microorganisms causing the diseases histoplasmosis and paracoccidioidomycosis. This example would not solely be limited to the use of this type of control.

Another aspect of the present invention is a kit comprising pairs of primers capable of amplifying, simultaneously and in a single reaction, by means of the PCR technique, a fragment of the ITS region of the ribosomal DNA of *Histoplasma capsulatum* and/or *Paracoccidioides brasiliensis.*

The kit may include reagents for the amplification of a fragment of the ITS region of the ribosomal DNA of *Histoplasma capsulatum* and *Paracoccidioides brasiliensis.* The reagents included in this kit may be other specific primers of the ITS nucleotide region, specific probes marked with two different fluorophores as well as with a quencher, as described in this specification.

The kit may also include positive and negative controls. Positive and negative controls are understood to be components of the kit that make it possible to check the efficacy of the reagents supplied therein. The nature of the controls depends of the nature of the reagents, hence, a positive control may be, for example, a genetic construction that includes the sequences of the fragments of the ITS object of study in the present invention, which may be amplified by the pairs of primers described above and the negative control may be a construction lacking the fragments of ITS present in the positive control. Other positive and negative controls may be included in this kit to assess the functioning thereof.

In a preferred embodiment, the kit comprises the primers SEQ ID NO: 1 and SEQ ID NO: 2 for amplifying a fragment of the ITS2 region of the ribosomal DNA of *Histoplasma capsulatum* and the primers SEQ ID NO: 4 and SEQ ID NO: 5 for amplifying a fragment of the ITS1 region of the ribosomal DNA of *Paracoccidioides brasiliensis,* by means of PCR.

In another preferred embodiment of the kit, the amplification is performed by means of real-time multiplex PCR and probes marked with two different fluorophores are used.

In a more preferred embodiment of the kit, the sequences of the probes are SEQ ID NO: 3 for the amplified fragment of *Histoplasma capsulatum* and SEQ ID NO: 6 for the amplified fragment of *Paracoccidioides brasiliensis.* The probes are marked at the 5' end with a different fluorophore in each case and at the 3' end with the quencher BHQ1

In various preferred embodiments the kit is used for:
- diagnosis of histoplasmosis and/or paracoccidioidomycosis in a biological sample, environmental sample or in a microbiological culture.
- monitoring of the response to a treatment of histoplasmosis and/or paracoccidioidomycosis.

Throughout the description and the claims the word "comprises" and its variants are not intended to exclude other technical characteristics, additives, components or steps. For persons skilled in the art, other objects, advantages and characteristics of the invention will be inferred in part from the description and in part from the practice of the invention. The following figures and examples are provided by way of illustration, and are not intended to limit the present invention.

### DESCRIPTION OF THE FIGURES

**FIG 1****. Shows the ITS2 region of the ribosomal DNA of** *Histoplasma capsulatum.*
   The figure shows the internal position of ITS2 of *Histoplasma capsulatum.* The primers SEQ ID NO: 1 and SEQ ID NO: 2 amplify a fragment situated in ITS2, between the sequences that code for the ribosomal RNA 5.8S and 28S.
**FIG 2****. Shows the ITS1 region of the ribosomal DNA of** *Paracoccidioides brasiliensis.*
   The figure shows the internal position of ITS1 of *Paracoccidioides brasiliensis.* The primers SEQ ID NO: 4 and SEQ ID NO: 5 amplify a fragment situated in ITS2, between the sequences that code for the ribosomal RNA 18S and 5.8S.

### EXAMPLES

### EXAMPLE 1

### Design of the real-time PCR technique.

Ribosomal DNA-directed initiators and probes of the *Molecular Beacon* type specific for *H. capsulatum* and *P. brasiliensis* were designed, in particular to the ITS2 region (*Internal Transcriber Spacer* 2) in the case of *H. capsulatum* and to the ITS1 region in the case of *P. brasiliensis.*

For the design of the initiators and of the probes, the sequences of the ITS regions (*Internal Transcriber Spacers*) of the ribosomal DNA of 20 strains of *H*. *capsulatum* and of a strain of *P. brasiliensis* available in the Mycology Service were analysed as well as 14 clinical strains of *P. brasiliensis* from the *Instituto de Pesquisa Clinica Evandro Chagas.* The design of the initiators and of the probes was performed with the aid of the probe design program *Beacon Design* 5.0 (*Premier Biosoft,* Palo Alto, CA, USA). In the case of *H. capsulatum,* the direct initiator was SEQ ID NO: 1 and the reverse was SEQ ID NO: 2. The *Molecular Beacon* probe, defined by the sequence SEQ ID NO: 3, was marked at the 5' end with the fluorophore FAM and at the 3' end with the quencher or extinguisher BHQ1. In the case of *P. brasiliensis,* the direct initiator was SEQ ID NO: 4 and the reverse was SEQ ID NO: 5. The probe, defined by the sequence SEQ ID NO: 6, was marked at the 5' end with the fluorophore HEX and at the 3' end with the quencher BHQ1.

Table 1 contains the excitation and emission spectrums of two fluorochromes used in marking the probes.

**Table 1**

| **Fluorochrome** | **λ(nm)** | |
|---|---|---|
| | Excitation | Emission |
| FAM | 483 | 533 |
| HEX | 523 | 568 |

Once the initiators and the probes were designed, a search of BLAST type was carried out in the GenBank database (http://www.ncbi.nih.gov/Genbank/) to be sure that there was no homology with other microorganisms. Additionally, the specificity of the probes and the initiators designed by means of phylogenetic analysis of their sequences was verified. For these analyses the sequence database of the Mycology Service was used, which has 3600 strains belonging to 300 fungal species and the *Fingerprinting II informatix software,* version 3.0 (BIORAD, Madrid, Spain).

In a first stage of tuning of the technique the DNA detection of *H. capsulatum* and *P. brasiliensis* was optimized separately. The sensitivity and reproducibility of each PCR assay was determined separately and a study was also performed to know the specificity of the sequences chosen for the initiators and the probes. In this specificity study, two strains belonging to fungal species were used that cause infections similar to histoplasmosis and paracoccidioidomycosis and fungal species that frequently cause infections. These species were *Coccidioides immitis, Paracoccidioides brasiliensis* (included in the case of real-time PCR to detect *H. capsulatum*), *Histoplasma capsulatum* (included in the case of real-time PCR to detect *P. brasiliensis*) *Blastomyces dermatitidis, Aspergillus fumigatus, Aspergillus flavus, Aspergillus terreus, Fusarium solani, Fusarium verticillioides, Fusarium oxysporum, Scedosporium prolificans, Scedosporium apiospermum* and *Candida albicans.* Human and mouse genomic DNA was also included in the experiment (Promega, Madrid, Spain).

In a second stage the real-time multiplex PCR was optimized so that no decrease in sensitivity was produced with respect to individually standardized real-time PCRs. Since the fluorochromes were separated only 30 nm, a colour compensation was performed to avoid overlapping in fluorescence detection. To perform the colour compensation, a fluorescence reading was performed in both channels using the probes in a concentration of 0.4 µM. Once the conditions were standardized, said colour compensation was applied to each one of the experiments.

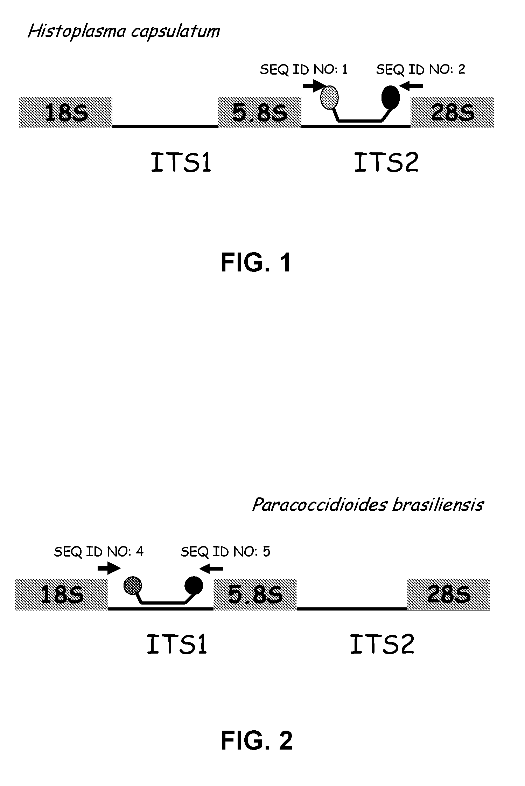

FIG 1 and FIG 2 schematically represent the areas of amplification chosen in each case.

### EXAMPLE 2

### in vitro validation of the detection method.

The 20 strains of *H. capsulatum,* the 15 strains of *P. brasiliensis* and the 11 strains of other species described in the previous paragraph were used. The extraction of nucleic acids was performed following habitual processes. In the case of the primary pathogens, the extraction was performed in a biosecurity laboratory for group 3 pathogens, meeting the established standards (Royal Decree 664/1997 from Spain).

The real-time PCR reactions contain a final volume of 20 µL, with 4.5mM of C12Mg, 0.5 µM of each initiator and 0.2 µM of the probe SEQ ID NO: 3 and *0.6* µM of probe SEQ ID NO: 6, furthermore, 2 µl of DNA were added to the PCR mixture. With respect to the PCR reaction conditions, a preincubation at 95ºC was performed followed by 50 denaturing cycles (25s at 95ºC), ringing (30s at 53ºC), and extension (5s at 72ºC). Finally, the protocol included a cycle to know the melting temperature of the double-stranded DNA of the amplified product (*melting curve*). This cycle is performed in the time interval from 30 to 80ºC. This cycle is always performed in the real-time PCR (or quantitative), since it helps to identify the amplified product, since the melting temperature is specific for each sequence.

The PCR reactions are performed in the LC480 equipment (*Roche Applied Science,* Madrid, Spain) and negative controls will be used in each experiment.

The technique reproducibility study, as well as the quantification of the amplified DNA is performed by means of the construction of regression lines with the results of five repetitions of different dilutions of DNA (from 10 ng to 1 fg DNA/µL) of *H. capsulatum* CNM-CM-2721 (Collection of filamentous fungi of the Mycology Centre of the National Microbiology Centre), as well as five repetitions of different dilutions of DNA of *P. brasiliensis* 20960 (Collection of filamentous fungi of the Mycology Centre of the National Microbiology Centre) (from 10 ng to 1 fg DNA/µL). The regression lines were made between the logarithms of the DNA concentrations and the PCR reaction cycle wherein the fluorescence was started to be detected (*crossing point, Ct*). The fluorescence was measured in the corresponding channels in each case. The reproducibility was obtained by means of the calculation of the coefficients of variation of the *Ct* for each of the DNA concentrations used. The regression lines made between the known concentrations of DNA and the *Ct* had a coefficient of determination (*r*2) of 0.99 (*P*<0.01). The test reproducibility was very high the average of the coefficients of variation being around 3% for *H. capsulatum* and in the case of *P*. *brasiliensis,* it was positioned around 4%.

The sensitivity was also high, the detection limit being between 10 fg and 1 fg per µL of sample analysed, although in the case of detection of *P. brasiliensis* in the lower concentrations (1 fg per µL), the reproducibility is lost, the reproducible limit being 10 fg per µL.

The specificity was 100%, since DNA from the other 11 species included in the studies was not detected in any of the reactions, nor murine or human DNA.

### EXAMPLE 3.

### Validation of the method for the identification and confirmation of colonies under culture.

46 strains were used numbered in the preceding sections. The same extraction and amplification techniques were used. The sensitivity was 100% and the specificity 100%. All these experiments were performed in the facilities with level 3 biosecurity measures.

### EXAMPLE 4

### Validation of the technique in biological samples

Once the multiplex PCR technique was standardized *in vitro* and its use was verified as an identification method of colonies under culture, an adaption thereof was performed, to use it in biological samples.

The DNA extraction from the biological samples was performed by means of habitual processes and also using the QiampDNA Mini Kit (Qiagen, Izasa, Madrid, Spain). 2 µl were used of the DNA extracted from each sample for each real-time PCR reaction. The only exception was the samples of serum verifying that the use of 4 µl improved test sensitivity.

In the case of paraffined samples, a previous step was performed to deparaffize consisting of washing with 1.2 ml of xylene followed by two washes with 1.2 ml of ethanol (96-100%). To validate the real-time PCR technique in biological samples, 44 samples were used from 20 patients, 18 of them with proven histoplasmosis and two patients with proven paracoccidioidomycosis. The infection had been confirmed by means of conventional diagnosis techniques such as culture, histological examination or positive serology, plus the clinical symptoms and the compatible epidemiological records. It must be highlighted that the PCR technique was applied to the available biological samples, which were sent from 20 different healthcare centres, depending on the symptoms, availability and possibilities of each case.

The PCR technique was positive in 14 of the 18 patients with histoplasmosis and in the two patients with paracoccidioidomycosis, which represents in total 80% of all patients. By samples, 21 serums were analysed (serial samples of serum were obtained from some patients), 11 samples related to the respiratory apparatus, 3 bone marrow aspirates, 3 plasma samples, 2 blood samples, two biopsies and a urine sample. The PCR technique showed 91% sensitivity in samples related to the respiratory apparatus, since it detected DNA of this species in 10 of the 11 samples analysed (bronchoalveolar lavage, bronchial secretions, sputum and oropharyngeal exudate). In other types of biological samples, the technique was positive in two of the three samples of bone marrow (66%) and in two of the three samples of plasma (66%) and in 100% of the biopsies. It was negative for the urine sample and for the three blood samples, probably due to the inhibition of the PCR by the components of said samples. With respect to the serums, it was positive in 9 of the 21 serums (42.8%). Positive results were never obtained in the serums of patients with paracoccidioidomycosis but it is a result that can be expected since the quantity of DNA in said samples is very small. In total, the technique was positive in 25 of the samples analysed (57%).

The table summarizes the results of the samples analysed together with the serological determinations and the results of the culture, in the samples received.

**Table 2. Results of the real-time PCR techniques in 20 patients with histoplasmosis and two patients with paracoccidioidomycosis.**

| **Patient** | **Samples** | **PCR result** | **Culture result** | **Result of the immunodiffusion** |
|---|---|---|---|---|
| 1 | Serum | Negative | NP | Positive |
| 2 | Serum | Negative | NP | Positive |
| 3 | Serum | Positive | NP | Negative |
| | Blood | Negative | Positive | NP |
| 4 | Serum | Positive | NP | Positive |
| 5 | Serum 1 | Negative | NP | Negative |
| | Serum 2 | Negative | NP | Negative |
| 6 | Serum 1 | Positive | NP | Negative |
| | Serum 2 | Negative | NP | Negative |
| | Serum 3 | Negative | NP | Negative |
| | Serum 4 | Negative | NP | Negative |
| 7 | Serum 1 | Positive | NP | Positive |
| | Serum 2 | Positive | NP | Positive |
| 8 | Serum 1 | Negative | NP | Negative |
| | Serum 2 | Positive | NP | Negative |
| 9 | Bronchial secretion | Positive | Positive | NP |
| | Bronchoalveolar lavage | Positive | Positive | NP |
| | Bone marrow | Negative | Positive | NP |
| 10 | Bone marrow | Positive | Positive | NP |
| 11 | Sputum | Negative | Positive | NP |
| | Bronchoalveolar lavage | Positive | Positive | NP |
| | Urine | Negative | Negative | NP |
| 12 | Serum | Positive | NR | Positive |
| | Bronchial secretion | Positive | Positive | NP |
| | Bronchoalveolar lavage | Positive | Positive | NP |
| | Bone marrow | Positive | Positive | NP |
| 13 | Plasma | Negative | NP | NP |
| 14 | Serum 1 | Negative | NP | Negative |
| | Plasma 1 | Positive | NP | NP |
| | Serum 2 | Positive | NP | Negative |
| | Plasma 2 | Negative | NP | NP |
| 15 | Oropharyngeal ex. | Positive | Positive | NP |
| 16 | Bronchoalveaolar lavage | Positive | Positive | NP |
| 17 | Serum | Positive | NP | Positive |
| | Lymph node | Positive | NP | NP |
| 18 | Serum | Negative | NP | Negative |
| | Blood | Negative | NP | NP |
| | Biopsy | Positive | NP | NP |
| | Sputum | Positive | NP | NP |
| 21* | Serum | Negative | NP | Positive |
| | Sputum | Positive | NP | NP |
| | Biopsy | Positive | NP | NP |
| 22* | Serum | Negative | NP | Positive |
| | Sputum | Positive | NP | NP |

| | | | | |
|---|---|---|---|---|
| NP: Not performed | | | | |

The average of the DNA concentrations in biological samples was 51.36 fg /µl in the serum samples and 25.2 x10³ fg /µl in the other samples analysed, which can explain the greater sensitivity of the technique in non-serological samples.

A specificity study was also performed. 40 samples of serum were analysed from neutropenic patients (with decreased concentration of neutrophils in the blood), 10 of them with positive PCR for *Aspergillus fumigatus* and a further 30 with negative PCR. For all of them the real-time PCR technique specific multiple for *H. capsulatum* and *P. brasiliensis* was negative. Five serums were also analysed from healthy patients from endemic areas, also obtaining negative results.

These results indicate that the PCR technique demonstrated a considerable sensitivity in all samples analysed, particularly in the samples related to the respiratory apparatus. No cross reaction was detected between both pathogens, the negative technique being a histoplasma in the cases of paracoccidioidomycosis and vice-versa. Furthermore, the result of the PCR was obtained 24-48 hours after receiving the sample in the Mycology Service, which shows it is a rapid diagnosis technique.

In parallel the presence of antibodies was detected for *H. capsulatum* and *P*. *brasiliensis* in the serum samples and the blood sample culture was performed, related to the respiratory apparatus, of bone marrow and urine. As can be seen in the previous table, samples were cultured, a total of 12 being positive (52%). The culture was positive between 10 and 14 days after seeding. It also determined the anti *Histoplasma* and *Paracoccidioides* antibodies by means of an immunodiffusion technique (ID *Fungal Antibody System, Immuno-Mycologics,* Leti Laboratorios, Madrid, Spain), the technique being positive in nine serums of the 21 received (42%). The immunodiffusion was invariably negative in most of the samples from HIV+ patients or with other immunodepressions.

## Claims

1. A method for simultaneously detecting *Histoplasma capsulatum* and *Paracoccidioides brasiliensis* comprising:
a. obtaining a sample and isolating its DNA,
b. amplifying a fragment of the ITS region of the ribosomal DNA of *Histoplasma capsulatum* and/or *Paracoccidioides brasiliensis* contained in the DNA isolated in step (a),
c. determining the deviation of step (b) with respect to the controls and
d. analysing the deviation of step (c) and attributing it to the presence of said microorganisms.

2. The method according to claim 1 wherein the concentration of the DNA isolated to carry out the amplification is at least 10 fg/µl.

3. The method according to claim 1 wherein the sample is:
a. a biological sample,
b. a microbiological culture or
c. an environmental sample.

4. The method according to claim 3, where the biological sample is related to the respiratory apparatus.

5. The method according to any of claims 1 to 4, where the amplification of the ITS2 region of the ribosomal DNA of *Histoplasma capsulatum* is performed by means of the primers SEQ ID NO: 1 and SEQ ID NO: 2 and the amplification of the ITS1 region of the ribosomal DNA of *Paracoccidioides brasiliensis* is performed by means of the primers SEQ ID NO: 4 and SEQ ID NO: 5.

6. The method according to any of claims 1 to 5, where the amplification is performed by means of real-time multiplex PCR **characterized in that** specific probes are used marked with two different fluorophores.

7. The method according to claim 6 where the probes are:
a. SEQ ID NO: 3 for the amplified fragment of *Histoplasma capsulatum* and it is marked at the 5' end with a fluorophore and at the 3' end with a quencher, and
b. SEQ ID NO: 6 for the amplified fragment of *Paracoccidioides brasiliensis* and it is marked at the 5' end with a different fluorophore from that used in step (a) and at the 3' end with a quencher.

8. The method according to claim 7, wherein the fluorophore of step (a) is FAM, the fluorophore of step (b) is HEX and the quencher is BHQ1.

9. The method according to any of claims 1 to 8, for the monitoring of the response to a treatment of histoplasmosis and/or paracoccidioidomycosis.

10. A kit for simultaneously detecting *Histoplasma capsulatum* and *Paracoccidioides brasiliensis* comprising pairs of primers that can amplify, by means of the PCR technique, a fragment of the ITS region of the ribosomal DNA of *Histoplasma capsulatum* and/or *Paracoccidioides brasiliensis.*

11. The kit according to claim 10 comprising the primers SEQ ID NO: 1 and SEQ ID NO: 2 for amplifying a fragment of the ITS2 region of the ribosomal DNA of *Histoplasma capsulatum* and the primers SEQ ID NO: 4 and SEQ ID NO: 5 for amplifying a fragment of the ITS1 region of the ribosomal DNA of *Paracoccidioides brasiliensis.*

12. The kit according to any of claims 10 or 11, wherein the amplification is performed by means of real-time multiplex PCR and specific probes are used marked with two different fluorophores.

13. The kit according to claim 12, wherein the probes are:
a. SEQ ID NO: 3 for the amplified fragment of *Histoplasma capsulatum* and it is marked at the 5' end with a fluorophore and at the 3' end with a quencher, and
b. SEQ ID NO: 6 for the amplified fragment of *Paracoccidioides brasiliensis* and it is marked at the 5' end with a different fluorophore from that used in step (a) and at the 3' end with a quencher.

14. The kit according to claim 13, wherein the fluorophore of step (a) is FAM, the fluorophore of step (b) is HEX and the quencher is BHQ1.

15. The kit according to any of claims 10 to 14, for the diagnosis of histoplasmosis and/or paracoccidioidomycosis in a biological sample, environmental sample or in a microbiological culture.

16. The kit according to any of claims 10 to 14, for the monitoring of the response to a treatment of histoplasmosis and/or paracoccidioidomycosis.
